# EUROPÄISCHE PATENTANMELDUNG

(11) **EP 0 720 988 A1**
(43) Veröffentlichungstag der Anmeldung: **10.07.1996**
(21) Anmeldenummer: 95120371.0
(22) Anmeldetag: 22.12.1995
(51) Int. Cl.: C07K 5/02, C07D 209/02, A61K 38/55, A61K 31/395

(54) **Neue Pseudopeptide mit Trifluormethyl-substituiertem 2-Azabicyclooctan**

(30) Priorität: 04.01.1995 DE 19500122
(71) Anmelder: BAYER AG, 51368 Leverkusen (DE)
(72) Erfinder: Raddatz, Siegfried, Dr., D-51065 Köln (DE); Wild, Hanno, Dr., Orange, Connecticut 06477 (US); Häblich, Dieter, Dr., D-42115 Wuppertal (DE); Röben, Wolfgang, Dr., D-51467 Bergisch Gladbach (DE); Hansen, Jutta, Dr., D-42115 Wuppertal (DE); Paessens, Arnold, Dr., D-42781 Haan (DE)

(57) **Zusammenfassung**

Die vorliegende Erfindung betrifft neue Pseudopeptide mit trifluoromethyl-substituiertem 2-Azabicyclooctan der allgemeinen Formel (I)

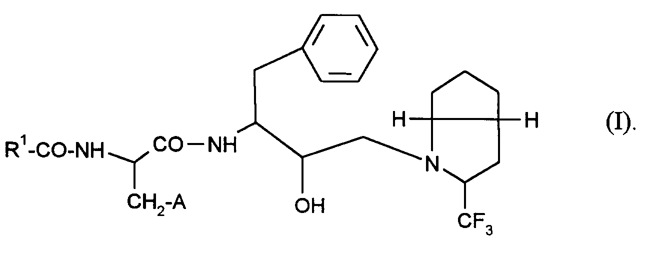

## Beschreibung

Die vorliegende Erfindung betrifft neue Pseudopeptide mit Trifluoromethyl-substituiertem 2-Azabicyclooctan, ein Verfahren zu ihrer Herstellung und ihre Verwendung als antiretrovirale Mittel.

Außerdem sind in der EP 528 242 Trifluoromethyl-haltige Pseudopeptide als antiretrovirale Mittel beschrieben, wobei einige der hier aufgeführten erfindungsgemäßen Verbindungen vom breitesten Bedeutungsumfang dieser Publikation umfaßt sind.

Die vorliegende Erfindung betrifft neue hochwirksame Pseudopeptide mit trifluoromethyl-substituiertem 2-Azabicyclooctan der allgemeinen Formel (I) 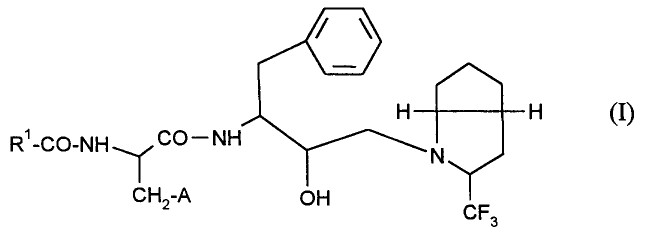
in welcher
- A: für Cyano oder für einen Rest der Formel -CO-NH₂ steht,
- R¹: für einen Rest der Formel 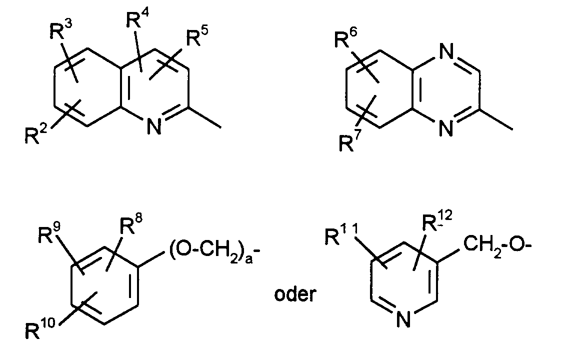 steht,
worin
- R², R³, R⁴, R⁵, R⁶, R⁷, R⁸, R⁹, R¹⁰, R¹¹ und R¹²: gleich oder verschieden sind und Phenyl, Hydroxyl, Wasserstoff, Halogen, geradkettiges oder verzweigtes Alkyl mit bis zu 4 Kohlenstoffatomen, Morpholinyl, Phenylthio oder Cycloalkyl mit 3 bis 6 Kohlenstoffatomen bedeuten,
- a: eine Zahl 0 oder 1 bedeutet,
und deren Salze.

Die erfindungsgemäßen Verbindungen der allgemeinen Formel (I) haben mehrere asymmetrische Kohlenstoffatome.

Der Rest der allgemeinen Formel (A) 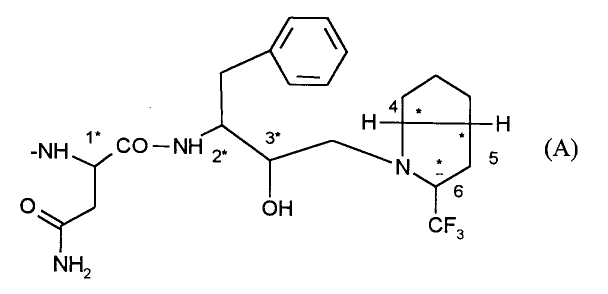
besitzt 6 asymmetrische Kohlenstoffatome (*), die unabhängig voneinander in der R- oder S-Konfiguration vorliegen. Bevorzugt sind die Konfigurationen 1(R), 2(R), 3(S), 4(S), 5(S) und 6(S).

Physiologisch unbedenkliche Salze der Pseudopeptide mit trifluoromethyl-substituiertem 2-Azabicyclooctan können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Procain, Dibenzylamin, N-Methylmorpholin, Dihydroabietylamin, 1-Ephedrinamin oder Methyl-piperidin.

Bevorzugt sind Verbindungen der allgemeinen Formel (I),
in welcher
- A: für Cyano oder für einen Rest der Formel -CO-NH₂ steht,
- R¹: für einen Rest der Formel 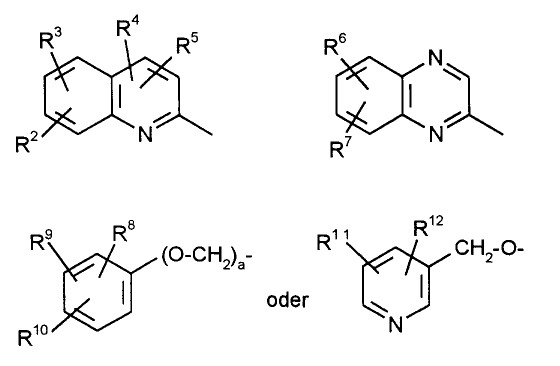 steht,
worin
- R², R³, R⁴, R⁵, R⁶, R⁷, R⁸, R⁹, R¹⁰, R¹¹ und R¹²: gleich oder verschieden sind und Phenyl, Hydroxyl, Wasserstoff, Fluor, Chlor, Brom, geradkettiges oder verzweigtes Alkyl mit bis zu 3 Kohlenstoffatomen, Morpholinyl, Phenylthio, Cyclopentyl oder Cyclohexyl bedeuten,
- a: eine Zahl 0 oder 1 bedeutet,
und deren Salze.

Besonders bevorzugt sind Verbindungen der allgemeinen Formel (I),
in welcher
- A: für Cyano oder für einen Rest der Formel -CO-NH₂ steht,
- R¹: für einen Rest der Formel 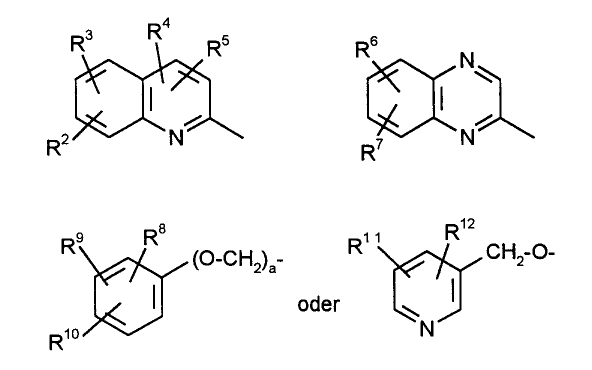 steht,
worin
- R², R³, R⁴, R⁵, R⁶, R⁷, R⁸, R⁹, R¹⁰, R¹¹ und R¹²: gleich oder verschieden sind und Wasserstoff, Phenyl, Hydroxyl, Fluor, Chlor, Brom, Methyl, Phenylthio, Cyclopentyl, Cyclohexyl oder Morpholinyl bedeuten,
- a: eine Zahl 0 oder 1 bedeutet,
und deren Salze.

Außerdem wurde ein Verfahren zur Herstellung der erfindungsgemäßen Verbindungen der allgemeinen Formel (I) gefunden, dadurch gekennzeichnet, daß man

Carbonsäuren der allgemeinen Formel (II)

R¹-CO₂H (II)

in welcher
- R¹: die oben angegebene Bedeutung hat
mit der Verbindung der Formel (III), 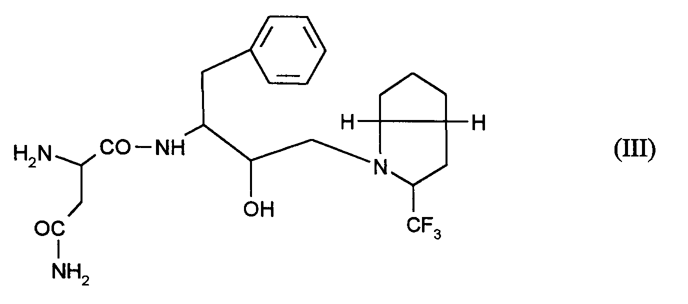
gegebenenfalls unter vorgeschalteter Aktivierung der Carbonsäure, in inerten Lösemitteln und in Anwesenheit einer Base und/oder eines Hilfsmittels umsetzt,
und im Fall A = CN abschließend mit Dimethylcarbamoylchlorid ebenfalls in einem der oben aufgeführten Lösemitteln umsetzt.

Das erfindungsgemäße Verfahren kann durch folgendes Formelschema beispielhaft erläutert werden: 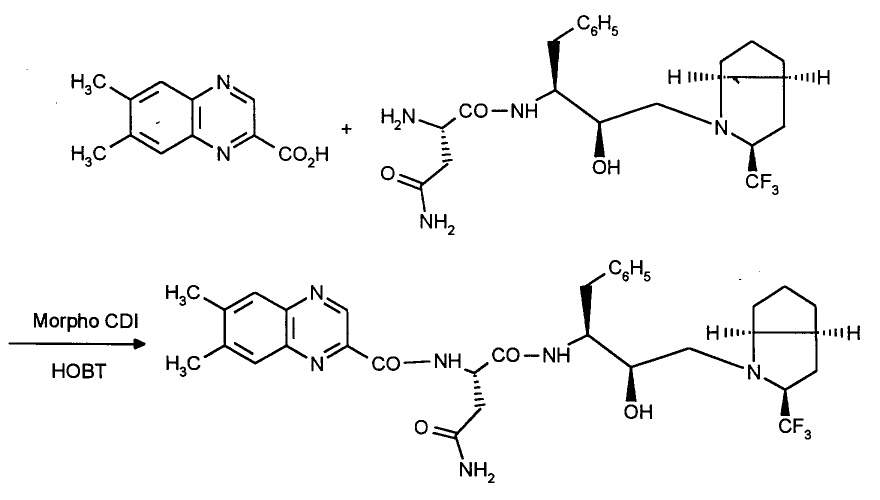
Als Lösemittel eignen sich für alle Verfahrensschritte die üblichen inerten Lösemittel, die sich unter den Reaktionsbedindungen nicht verändern. Hierzu gehören bevorzugt organische Lösemittel wie Ether z.B. Diethylether, Glykolmono- oder -dimethylether, Dioxan oder Teträhydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Dimethylformamid und Tetrahydrofuran.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridin oder N-Methylpiperidin, oder Amide wie Natriumamid oder Lithiumdiisopropylamid, oder Lithium-N-silylalkylamide, wie beispielsweise Lithium-N-(bis)triphenysilylamid oder Lithiumalkyle wie n-Butyllithium.

Die Base wird in einer Menge von 1 Mol bis 10 Mol, bevorzugt von 1 Mol bis 3 Mol, bezogen auf 1 Mol der Verbindungen der allgemeinen Formel (II), eingesetzt.

Als Hilfsmittel eignen sich bevorzugt Kondensationsmittel, die auch Basen sein können, insbesondere wenn die Carboxylgruppe als Anhydrid aktiviert vorliegt. Bevorzugt werden hier die üblichen Kondensationsmittel wie Carbodiimide, z.B. N,N'-Diethyl-, N,N'-Diisopropyl-, N,N'-Dicyclohexylcarbodiimid, N-(3-Dimethylaminoisopropyl)-N'-ethyl-carbodiimid-Hydrochlorid, N-Cyclohexyl-N'-(2-morpholinoethyl)-carbodiimid-metho-p-toluolsulfonat (CMCT bzw. Morpho CDI), oder Carbonylverbindungen wie Carbonyldiimidazol, oder 1,2-Oxazoliumverbindungen wie 2-Ethyl-5-phenyl-1,2-oxazolium-3-sulfat oder 2-tert.Butyl-5-methyl-isoxazolium-perchlorat, oder Acylaminoverbindungen wie 2-Ethoxy-1-ethoxycarbonyl-1,2-dihydrochinolin, oder Propanphosponsäureanhydrid, oder Isobutylchloroformat, oder Benzotriazolyloxy-tri(dimethylamino)phosphonium-hexafluorophosphat oder 1-Hydroxybenzotriazol.

Die Reaktionen können sowohl bei Normaldruck als auch bei erhöhtem oder erniedrigtem Druck (beispielsweise 0,5 bis 5 bar), bevorzugt bei Normaldruck, durchgeführt werden.

Die Umsetzung erfolgt im allgemeinen in einem Temperaturbereich von -20°C bis +40°C, bevorzugt von 0°C bis Raumtemperatur.

Die Umsetzung mit Dimethylcarbamoylchlorid erfolgt vorzugsweise in Pyridin und unter Schutzgasatmosphäre.

Die Reaktion wird im allgemeinen in einem Temperaturbereich von +80°C bis +120°C, vorzugsweise bei 100°C durchgeführt.

Die Carbonsäuren der allgemeinen Formel (II) sind im Fall der Chinoline, Phenyle, Pyridyle und Biphenyle an sich bekannt. Die Chinoxalin-Carbonsäuren sind teilweise bekannt oder neu und können dann beispielsweise durch Umsetzung der entsprechenden Phenylendiamine mit dem Oson der D-Fructose - in situ hergestellt durch Umsetzung des Phenylhydrazons mit NaNO₂ und Salzsäure - und anschließender Oxidation mit H₂O₂-Lösung hergestellt werden [vgl. hierzu G. Henseke, Chem. Ber. 91, 1958, 1605-11 und DE 24 10 852].

Die Verbindung der allgemeinen Formel (III) ist neu und kann beispielsweise hergestellt werden, indem man

Verbindungen der allgemeinen Formel (IV) 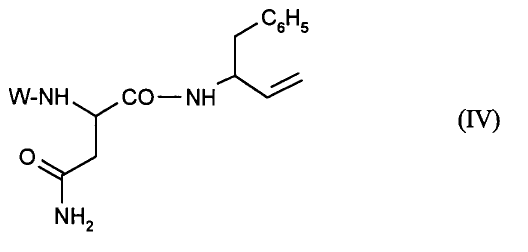
in welcher
- W: für eine Aminoschutzgruppe, vorzugsweise tert.Butoxycarbonyl,
steht,
zunächst mit einer Epoxidierungsreaktion, gegebenenfalls mit Hilfe einer Base oder durch eine Phasentransferkatalyse, in die Verbindungen der allgemeinen Formel (V), 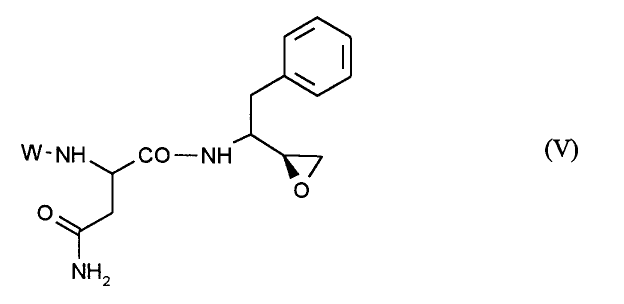
in welcher
- W: die oben angegebene Bedeutung hat,
überführt und anschließend mit 3-Trifluormethyl-2-azabicyclo[3.3.0]octan der Formel (VI) 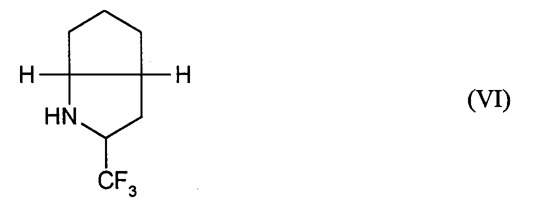
in Lösemitteln umsetzt und die Schutzgruppe nach üblichen Methoden abspaltet.

Als Lösemittel eignen sich die üblichen organischen Lösemittel, die sich unter den Reaktionsbedingungen nicht verändern. Hierzu gehören bevorzugt organische Lösemittel wie Alkohole, z.B. Methanol, Ethanol oder n-Propanol, Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Dichlorethan (DCE), Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Dichlorethan, Dimethylformamid und n-Propanol.

Als Reagentien für die Epoxidierung eignen sich die literaturbekannten Verbindungen wie beispielsweise m-Chlorbenzoesäure, Magnesiummonoperoxyphthalat, Dimethyldioxiran oder Methyl(trifluormethyl)dioxiran. Bevorzugt sind m-Chlorperbenzoesäure und Magnesiummonoperoxyphthalat [vgl. P. Brongham et al., Synthesis (1987), 1015; W. Adam et al., J. Org. Chem 52, 2800 (1987) und R. Curci et al., J. Org. Chem. 53, 3890 (1988)].

Wird die Epoxidierung mit Hilfe einer Phasentransfer-Katalyse durchgeführt, so werden als Hilfsstoffe beispielsweise organische Ammoniumchloride oder -bromide wie beispielsweise Benzyltriethylammoniumchlorid oder -bromid, Methyltrioctylammoniumchlorid, Tetrabutylammoniumbromid, Tricaprylmethylammoniumchlorid (Aliquat 336) eingesetzt. Bevorzugt sind Benzyltriethylammoniumchlorid und -bromid.

Die Epoxidierung wird in einem Temperaturbereich von -10°C bis +90°C, bevorzugt von 0°C bis +60°C, durchgeführt.

Die Reaktionen können sowohl bei Normaldruck als auch bei erhöhtem oder erniedrigtem Druck (beispielsweise 0,5 bis 5 bar), bevorzugt bei Normaldruck, durchgeführt werden.

Die Verbindungen der allgemeinen Formeln (IV) und (V) sind größtenteils bekannt [vgl. EP 528 242].

Die Verbindung der allgemeinen Formel (VI) ist neu und kann beispielsweise durch Fluorierung von 2-Azabicyclo[3.3.0]octan-3-carbonsäure in der jeweiligen Konfiguration mit HF und SF₄ hergestellt werden.

Es wurde überraschend gefunden, daß die Verbindungen der allgemeinen Formel (I) eine außerordentlich starke Wirkung gegen Retroviren besitzen. Dies wird mit einem HIV-spezifischen Protease-Enzymtest belegt.

Die Ergebnisse der unten aufgeführten Beispiele wurden nach dem in den folgenden Literaturangaben [vgl. Hansen, J., Billich, S., Schulze, T., Sukrow, S. and Mölling, K. (1988), EMBO Journal, Vol, 7, No. 6, pp. 1785 - 1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Die angegebenen IC₅₀-Werte beziehen sich auf die Substanzkonzentration, die unter den oben aufgeführten Testbedingungen eine 50%ige Hemmung der Protease-Aktivität bewirkt.

### Enzym assay, HIV-1

**Tabelle I**

| **Beispiel-Nr.** | **HIV-1-Protease-Inhibition** | |
|---|---|---|
| | **IC**_{**50**} **[Mol/l]** | **IC**_{**95**} **[Mol/l]** |
| 1 | 1,8 x 10⁻¹¹ | 1,9 x 10⁻¹⁰ |
| 2 | 1,7 x 10⁻¹¹ | 3,6 x 10⁻¹¹ |
| 3 | 2,4 x 10⁻¹⁰ | 2,2 x 10⁻⁸ |
| 4 | 6,0 x 10⁻¹⁰ | 3,3 x 10⁻⁸ |
| 5 | 9,5 x 10⁻¹¹ | 2,1 x 10⁻⁹ |
| 6 | 9,6 x 10⁻⁸ | 3,3 x 10⁻⁷ |
| 7 | 1,3 x 10⁻⁷ | 3,2 x 10⁻⁷ |
| 8 | 2,4 x 10⁻⁷ | 3,2 x 10⁻⁶ |
| 9 | 1,2 x 10⁻¹⁰ | 2,6 x 10⁻⁹ |
| 10 | 9,6 x 10⁻¹⁰ | 3,1 x 10⁻⁷ |
| 11 | 1,6 x 10⁻¹¹ | 3,6 x 10⁻¹¹ |
| 12 | 1,1 x 10⁻⁹ | 3,7 x 10⁻⁹ |
| 13 | 1,9x 10⁻¹¹ | 2,5 x 10⁻¹⁰ |
| 14 | 3,5 x 10⁻⁹ | 1,8 x 10⁻⁷ |
| 15 | 2,2 x 10⁻⁹ | 2,9 x 10⁻⁸ |
| 16 | 2,0 x 10⁻⁷ | 2,7 x 10⁻⁶ |
| 17 | 1,9 x 10⁻⁶ | 3,7 x 10⁻⁵ |
| 19 | 1,4 x 10⁻¹¹ | |

Außerdem zeigten die erfindungsgemäßen Verbindungen Wirkung in Lentivirus infizierten Zellkulturen. Dies konnte am Beispiel des HIV-Virus gezeigt werden.

### HIV-Infektion in Zellkultur

Der HIV-Test wurde mit geringen Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phytohaemagglutinin (90 µg/ml) und Interleukin-2 (40 U/ml) stimuliert. Zur Infektion mit dem in-fektiösen HIV wurden PBL's pelletiert und das Zellpellet wurde anschließend in 1 ml HIV-Virusadsorptionslösung suspendiert und 1 Stunde bei 37°C inkubiert.

Die Virusadsorptionslösung wurde zentrifugiert und das infizierte Zellpellet in Wachstumsmedium aufgenommen, so daß 1 x 10⁵ Zellen pro ml eingestellt waren. Die derart infizierten Zellen wurden zu 1 x 10⁴ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten 2¹⁰fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 Syncytien, währen die unbehandelte Zellkontrolle keine Syncytien aufwies.

Die IC₅₀-Werte wurden als die Konzentration der behandelten und infizierten Zellen ermittelt, bei der 50% (ca. 10 Syncytien) der virusinduzierten Syncytien durch die Behandlung mit der erfindungsgemäßen Verbindung unterdrückt waren.

Es wurde nun gefunden, daß die erfindungsgemäßen Verbindungen HIV infizierte Zellen vor der virusinduzierten Zellzerstörung schützen.

### Zellkulturassay, PBL

| Tabelle II | |
|---|---|
| **Beispiel-Nr.** | **PBL IC**_{**50**} **[Mol/l]** |
| 1 | 8,17 x 10⁻⁸ |
| 2 | 3,85 x 10⁻⁸ |
| 3 | 3,5 x 10⁻⁸ |
| 4 | 2,25 x 10⁻⁸ |
| 5 | 6,4 x 10⁻⁸ |
| 6 | 5,8 x 10⁻⁸ |
| 7 | 5,5 x 10⁻⁷ |
| 8 | 1,2 x 10⁻⁹ |
| 9 | 2,4 x 10⁻⁸ |
| 10 | 8,0 x 10⁻⁸ |
| 11 | 7,55 x 10⁻⁸ |
| 12 | 7,74 x 10⁻⁸ |
| 13 | 7,3 x 10⁻⁸ |
| 14 | 6,0 x 10⁻⁷ |
| 19 | 6,74 x 10⁻⁷ |

Die erfindungsgemäßen Verbindungen stellen wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:
1.) Die Behandlung und Prophylaxe von menschlichen Retrovirusinfektionen.
2.) Für die Behandlung oder Prophylaxe von durch HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.
3.) Für die Behandlung oder die Prophylaxe einer HTLV-I oder HTLV-II Infektion.
4.) Für die Behandlung oder die Prophylaxe des AIDS-carrier Zustandes (AIDS-Überträger-Zustand).

Als Indikationen in der Tiermedizin können beispielsweise angeführt werden:
Infektionen mit
a) Maedivisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegerziekte Virus (bei Schafen)
e) infektiösem Virus der Anämie (des Pferdes)
f) Infektionen, verursacht durch das Katzenleukämievirus
g) Infektionen, verursacht durch das Virus der Katzen-Immundefizienz (FIV)
h) Infektionen, verursacht durch das Virus der Affen-Immundefizienz (SIV)
Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen in einer Konzentration von etwa 0,1 bis 99,5 Gew.-%, vorzugsweise von etwa 0,5 bis 95 Gew.-% der Gesamtmischung, vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe, vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels, sowie dem Zeitraum bzw. Intervall, innerhalb welchem die Verabreichung erfolgt.

Die erfindungsgemäßen Verbindungen sind Enzyminhibitoren und können als solche für alle Zwecke eingesetzt werden für die Enzyminhibitoren brauchbar sind. Beispielhaft ist hier zu nennen der Einsatz als Label für Affinitätschromatographie, die Verwendung als Hilfsmittel für die Aufklärung von Enzymstrukturen und Reaktionsmechanismen sowie der Einsatz als Reagenz für Diagnostika.

### Ausgangsverbindungen

### Beispiel I

### 6,7-Dimethyl-2-[D-arabo-tetrahydroxy-butyl]chinoxalin

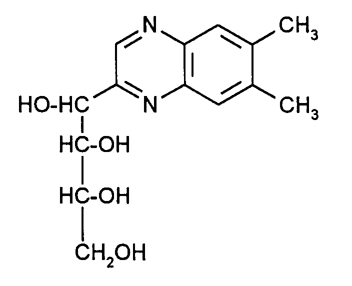

2,0 g (5,58 Mmol) D-Fructose-phenylosazon (hergestellt nach C.L. Butler, J. Am. Chem. Soc. 51, 1929, 3163) werden in 12 ml Wasser, 12 ml Ethanol und 1,4 ml konz. Salzsäure suspendiert, auf 45°C erwärmt und tropfenweise mit einer Lösung von 0,8g (11,2 Mmol) NaNO₂ in 2,5 ml Wasser versetzt. Man erhält eine dunkelrote, klare Lösung, die bei Raumtemperatur mit Natriumacetat abgepuffert wird. Man rührt alles mit 15 ml Essigester aus, trennt die dunkelrote, organische Phase ab, engt die gelbe, wäßrige Phase etwas ein und versetzt mit 0,5 g (3,67 Mmol) 4,5-Dimethylphenylendiamin. Man erhitzt das Gemisch 20 Minuten auf dem siedenden Wasserbad und filtert dann den gelben Niederschlag ab. Man rührt nun anschließend in etwas Methanol aus, filtriert erneut und trocknet.
Ausbeute: 0,3 g (29,4% d.Th.) gelbe Kristalle
Fp.: 190°C (Zers.)
DC: R_{f} (Substanz ist sehr schwer löslich) = 0,4 (Dichlormethan:Methanol= 9:1)

### Beispiel II

### 6,7-Dimethylchinoxalin-2-carbonsäure

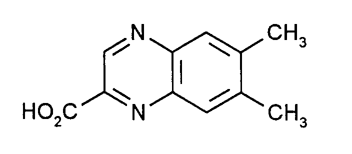

0,3 g (1,08 Mmol) der Verbindung aus Beispiel I werden in 10 ml 10%iger H₂O₂-Lösung suspendiert. Unter Rühren versetzt man mit 0,6 g (15 Mmol) NaOH (fest). Dabei beobachtet man ein Aufschäumen. Nach ca. einer Stunde Rühren erhält man eine klare Lösung. Beim Ansäuern mit konz. Salzsäure fällt ein farbloser Niederschlag aus. Er wird filtriert, mit Wasser gewaschen und getrocknet.
Ausbeute: 160 mg (73,3% d.Th.) farblose Kristalle
Fp.: 203°C (Zers.)
DC: R_{f} = 0,5 (Dichlormethan/Eisessig/Methanol 90/10/2)
In Analogie zu den Vorschriften der Beispiele I und II werden die folgenden substituierten Chinoxalin-2-carbonsäuren der Tabelle I hergestellt:

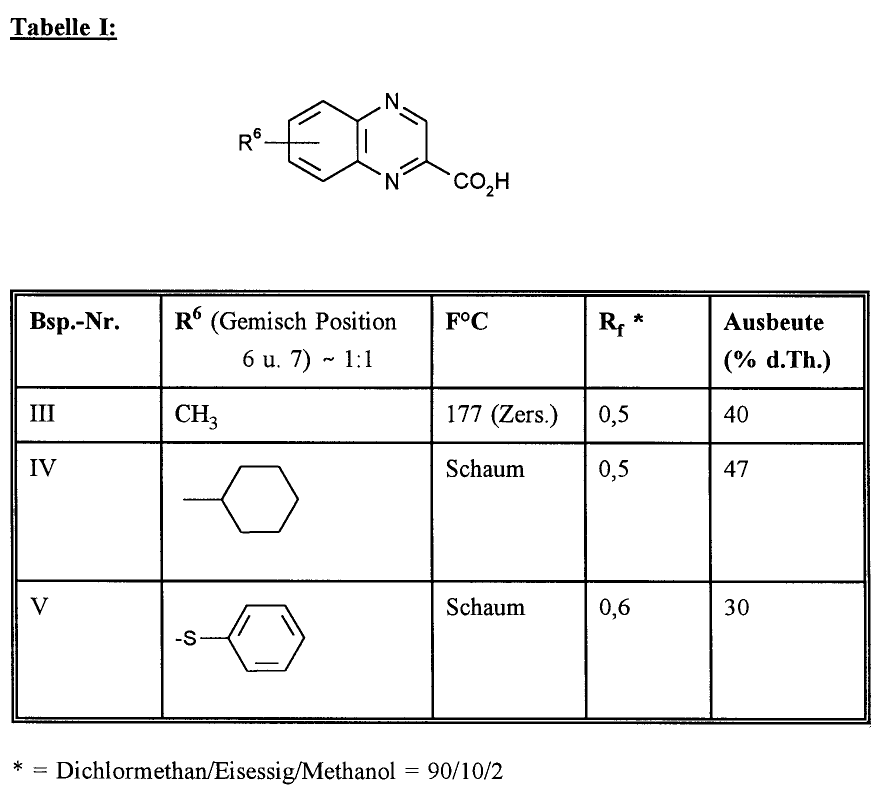

### Beispiel VI

### 3-Methyl-4,7-dichlorchinolin-2-carbonsäure-ethylester

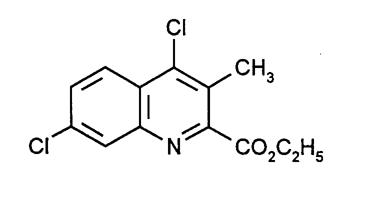

5 g (0,0187 Mol) 3-Methyl-4-hydroxy-7-chlorchinolin-2-carbonsäureethylester werden unter Argon mit 25 ml POCl₃ zwei Stunden in einem 140°C heißen Ölbad erhitzt. Anschließend destilliert man i.V. POCl₃ ab und übergießt vorsichtig mit Eis-wasser. Der ausgefallene Niederschlag wird abfiltriert, mit Wasser gewaschen, getrocknet und aus Dichlormethan / Cyclohexan (1/1) rekristallisiert.
Ausbeute: 3,8 g (71,0% d.Th.) farblose Kristalle
Fp.: 104°C
R_{f} (Dichlormethan) ~ 0,9

### Beispiel VII

### 3-Methyl-4-morpholinyl-7-chlorchinolin-2-carbonsäure-ethylester

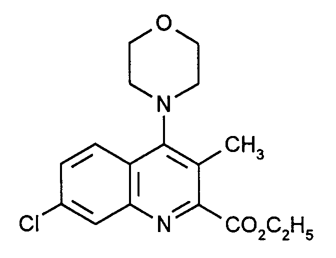

1 g (3,5 Mmol) der Verbindung aus Beispiel VI und 0,4 g (4,5 Mmol) Morpholin werden mit 2 Tropfen konz. HCl versetzt und in 5 ml abs. Ethanol 6 Stunden unter Rückfluß erhitzt. Es erfolgt keine Umsetzung. Nach Zusatz von 1 g (11,25 Mmol) Morpholin erhitzt man noch weitere 12 Stunden. Man engt alles i.V. ein und trennt den Rückstand säulenchromatographisch (Kieselgel 60, Dichlormethan / Essigester = 100/5).
Ausbeute: 100 mg (8,5% d.Th.) farblose Kristalle
Fp.: 74°C
R_{f} (Dichlormethan/Essigester = 100/3) = 0,45

### Beispiel VIII

### 3-Methyl-4-morpholinyl-7-chlorchinolin-2-carbonsäure

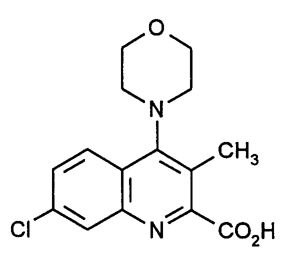

0,2 g (0,6 Mmol) der Verbindung aus Beispiel VII werden in 5 ml Ethanol gelöst, auf 50°C erwärmt, mit 1 ml 1 n Natronlauge versetzt und 10 Minuten bei 50°C gerührt. Nach dem Abkühlen versetzt man mit 1 ml 1 n Salzsäure und erhält einen fahlgelben Niederschlag. Er wird abfiltriert und getrocknet.
Ausbeute: 130 mg (70% d.Th.) hellgelbe Kristalle
Fp.: 196°C
R_{f} (Dichlormethan / Methanol = 9/1) = 0,5

### Beispiel IX

### (S,S,S)-3-Trifluormethyl-2-azabicyclo[3.3.0]octan

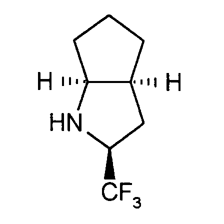

55 g (0,355 Mmol) (S,S,S)-2-Azabicyclo[3.3.0]octan-3-carbonsäure werden mit HF und SF₄ fluoriert. Die dunkelbraune, alkalische Suspension (ca. 200 ml) wird filtriert, i.V. auf ca. 50 ml eingeengt und dreimal mit 50 ml Dichlormethan extrahiert. Die organische Phase wird getrocknet, i.v. auf ein kleines Volumen eingeengt (dunkelbraunes Öl) und säulenchromatographisch getrennt (Kieselgel 60, Dichlormethan).
Ausbeute: 28,8 g (45,3% d.Th.) leicht bräunliches Öl
Fp.: um 0°C, GC: 99%
R_{f} = 0,4 (Dichlormethan)

### Beispiel X

### (2R,3S)-3-(N-Benzyloxycarbonyl)amino-1-{(S,S-S)-3-trifluormethyl-2-azabicyclo[3.3.0]octan-2-yl}-2-hydroxy-4-phenylbutan

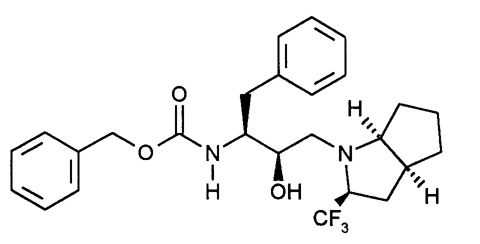

0,3 g (1 Mmol) (2R)-[1-(N-Benzyloxycarbonyl)amino-2-phenyl-(1S)-ethyl]oxiran und 0,15 g (0,84 Mmol) der Verbindung aus Beispiel IX werden in 2 ml 2-Propanol gelöst und 12 h in einem geschlossenen Gefäß in einem 130°C heißen Ölbad gerührt. Nach dem Erkalten engt man den Inhalt auf ein kleines Volumen ein und trennt ihn säulenchromatographisch (Kieselgel 60, Toluol/Essigester = 100/5).
Ausbeute: 72 mg (18% d.Th.) farbloser Schaum
R_{f} = 0,2 (Toluol / Essigester = 100/5)

### Beispiel XI

### (2R,3S)-3-Amino-1-{(S,S,S)-3-trifluormethyl-2-azabicyclo[3.3.0]octan-2-yl}-2-hydroxy-4-phenylbutan

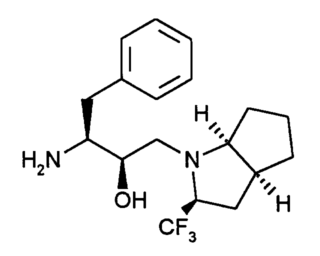

112 mg (0,235 Mmol) der Verbindung aus Beispiel X werden in 15 ml Methanol/THF (1:1) gelöst, mit etwas Pd/C (10%ig) versetzt und zwei Stunden bei Raumtemperatur hydriert. Nach Abfiltrieren des Katalysators und Abdampfen des Lösemittels erhält man ein leicht gelbes Öl.
Ausbeute: 80 mg (~quantitativ)
R_{f} = 0,6 (Dichlormethan / Methanol = 100/5)

### Beispiel XII

### (2R,3S)-3-[N-Benzyloxycarbonyl-L-asparaginyl)amino]-1-{(S,S,S)-3-trifluormethyl-2-azabicyclo[3.3.0]octan-2-yl}-2-hydroxy-4-phenyl-butan

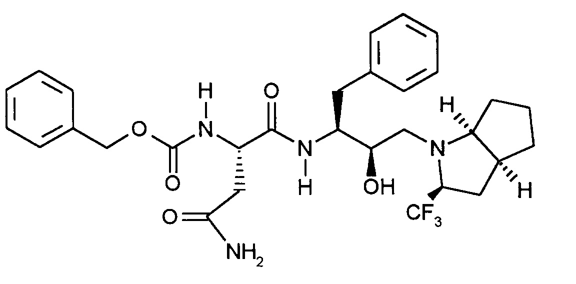

0,074 g (0,28 Mmol) Z-L-Asn-OH und 0,038 g (0,28 Mmol) HOBT (Hydroxybenzotriazol) werden unter Argonatmosphäre in 5 ml DMF gelöst, im Eisbad auf 0°C abgekühlt und unter Rühren mit 0,119 g (0,28 Mmol) Morpho-CDI versetzt. Man läßt 3 Stunden bei 0°C rühren und versetzt dann mit 0,08 g (0,234 Mmol) der Verbindung aus Beispiel XI, gelöst in 3 ml DMF. Man läßt die Temperatur auf RT ansteigen und rührt weiter über Nacht. Nach Zusatz von Wasser fällt ein farbloser Niederschlag an. Er wird filtriert, mit Wasser gewaschen und getrocknet.
Ausbeute: 87 mg (63,0 % d.Th.) farblose Kristalle
Fp.: 162°C
HPLC: 97,53 %ig

### Beispiel XIII

### (2R,3S)-3-(L-Asparaginyl)amino-1-(S,S,S)-3-trifluormethyl-2-azabicyclo[3.3.0]octan-2-yl}-2-hydroxy-4-phenylbutan

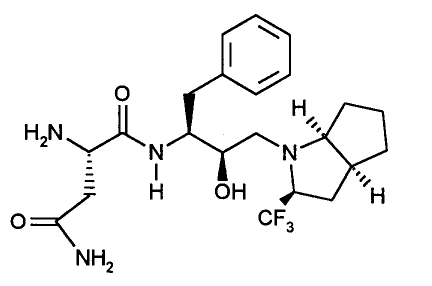

0,008 g (0,135 Mmol) der Verbindung aus Beispiel XII werden in 10ml Methanol/THF (1:1) gelöst, mit etwas Pd/C (10%ig) versetzt und bei RT 2 Stunden hydriert. Nach Abfiltrieren des Katalysators und Abdampfen des Lösemittels i.V. erhält man ein farbloses Öl.
Ausbeute: 58 mg (95% d.Th.)
R_{f} = 0,2 (Dichlormethan/Methanol = 100/5) (Ansprühen mit Ninhydrin)

### Herstellungsbeispiele

### Beispiel 1

### (2R,3S)-3-(Chinoxalin-2-yl-L-asparaginyl)amino-1-({(S,S,S)-3-trifluormethyl-2-azabicyclo[3.3.0]octan-2-yl}-2-hydroxy-4-phenylbutan

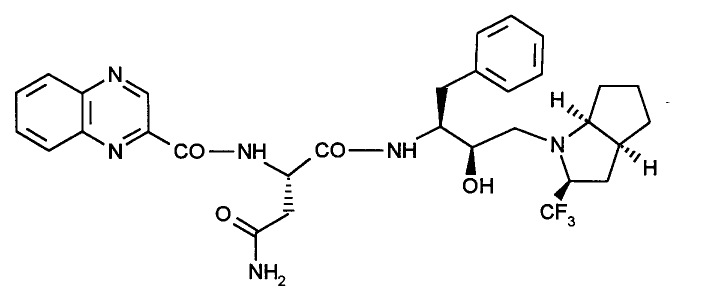

0,095 g (6,7 Mmol) HOBT und 0,071 g (0,7 Mmol) Triethylamin werden unter Argonatmosphäre in 2 ml DMF gelöst, auf -40°C abgekühlt und unter Rühren tropfenweise mit 0,14 g (0,7 Mmol) Chinoxalin-2-carbonsäurechlorid in 3 ml DMF versetzt. Man läßt 2 Stunden im Eisbad reagieren und versetzt dann mit 0,3 g (0,657 Mmol) der Verbindung aus Beispiel XIII. Man laßt das Gemisch über Nacht rühren, wobei die Temperatur auf Raumtemperatur ansteigt. Anschließend fällt man das gewünschte Produkt mit Wasser und reinigt es säulenchromatographisch (Kieselgel 60, Laufmittel: Dichlormethan/Methanol = 100/5).
Ausbeute: 130 mg (≙ 32,3% d.Th.) farbloser Schaum
R_{f} = 0,5 (Dichlormethan / Methanol = 100/5)

### Beispiel 2

### (2R,3S)-3-(Chinoxalin-2-yl-L-asparaginyl)amino-1-({(S,S,S)-3-trifluormethyl-2-azabicyclo[3.3.0]octan-2-yl}-2-hydroxy-4-phenylbutan Hydrochlorid

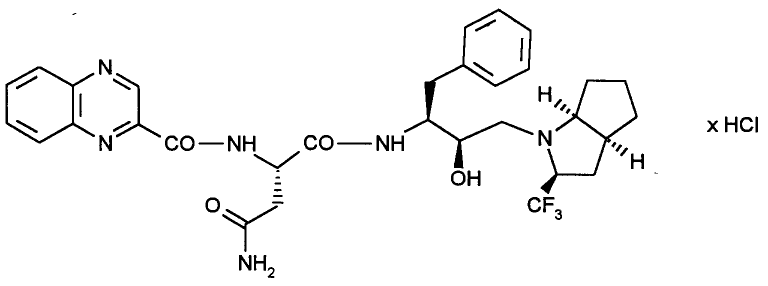

87 mg (0,142 Mmol) der Verbindung aus Beispiel 1 werden in 1 ml Methanol gelöst, mit 1,42 ml 0,1 n Salzsäure (0,142 Mmol) versetzt und die klare Lösung lyophilisiert.
Ausbeute: 89 mg (96% d.Th.) farbloser Schaum

### Beispiel 3

### (2R,3S)-3-(Chinolin-2-yl-L-asparaginyl)-amino-1-[(S,S,S)-3-trifluormethyl-2-azabicyclo[3.3.0]octan-2-yl}-2-hydroxy-4-phenyl-butan

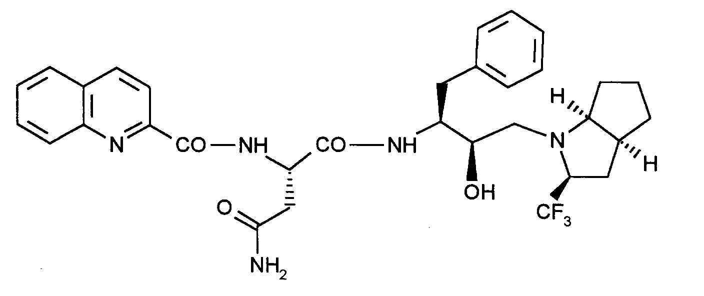

0,014 g (0,08 Mol) Chinolin-2-carbonsäure und 0,011 g (0,08 Mol) HOBT werden unter Argon in 5 ml DMF gelöst, im Eisbad auf 0°C abgekühlt und mit 0,034 g (0,08 Mol) Morpho-CDI versetzt. Nach 3stündigem Rühren bei 0°C gibt man 0,03 g (0,0657 Mol) der Verbindung aus Beispiel XIII gelöst in 1 ml DMF, hinzu und laßt über Nacht rühren, wobei die Temperatur auf Raumtemperatur ansteigt. Anschließend fällt man das gewünschte Produkt durch Wasserzusatz aus, filtriert, trocknet und reinigt es durch Ausfällen mit Methanol und Diisopropylether.
Ausbeute: 270 mg (67,2% d.Th.) farbloses Glas
R_{f} = 0,5 (Dichlormethan / Methanol = 100/5)

### Beispiel 4

### (2R,3S)-3-(Chinolin-2-yl-L-asparaginyl)-amino-1-[(S,S,S)-3-trifluormethyl-2-azabicylco[3.3.0]octan-2-yl}-2-hydroxy-4-phenyl-butan Hydrochlorid

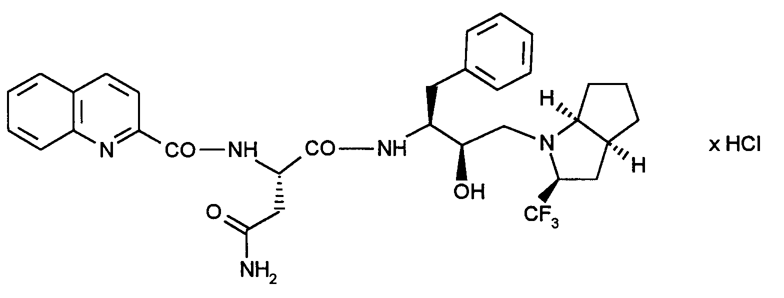

Die Titelverbindung wird in Analogie zur Vorschrift des Beispiels 2 aus 0,5 g (0,8175 Mmol) der Verbindung aus Beispiel 3 und 0,82 ml 0,1 n Salzsäure (0,82 Mmol) hergestellt.
Ausbeute: 0,5 g (96% d.Th.) farbloses Glas
In Analogie zu den obigen Beispielen werden die folgenden neuen Pseudopeptide mit trifluormethyl-substituiertem 2-Azabicyclooctan hergestellt, Tabelle 1:

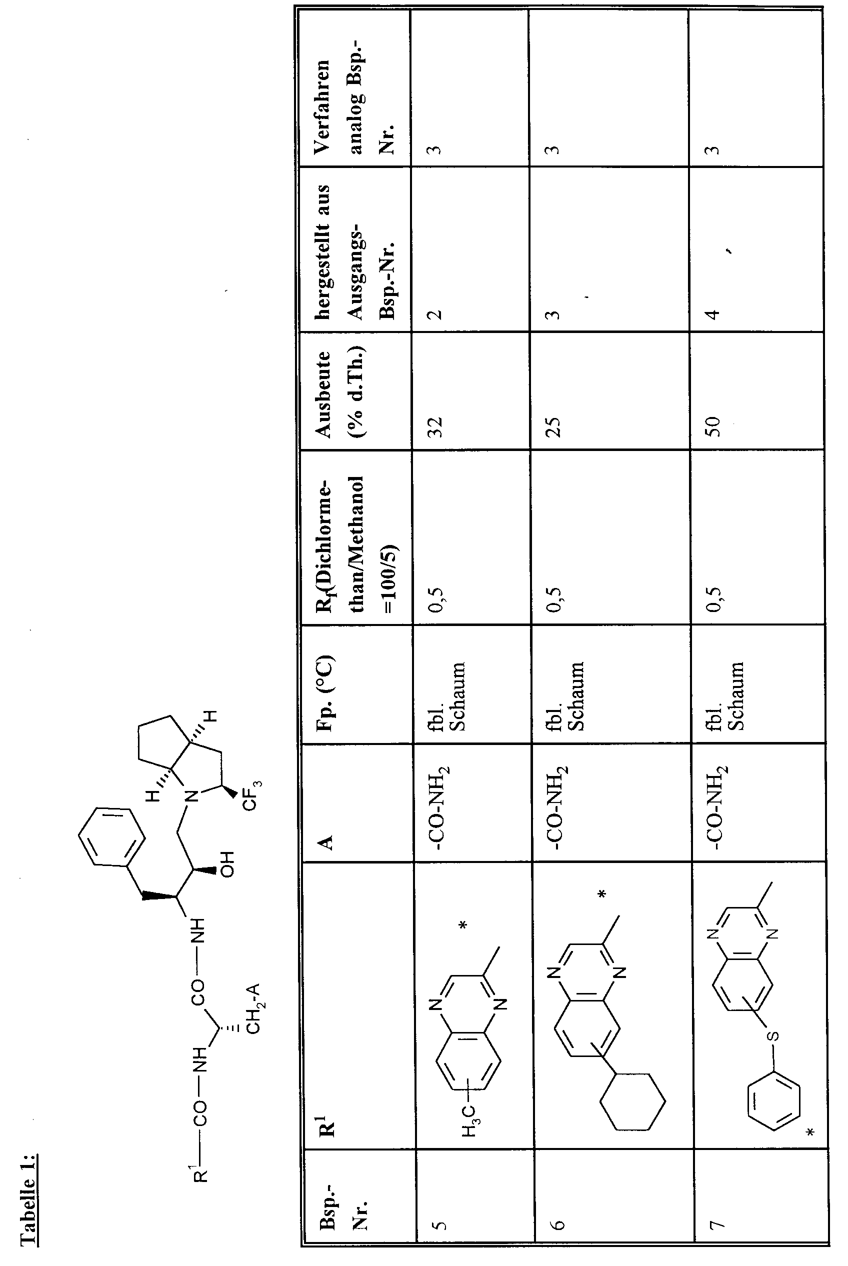

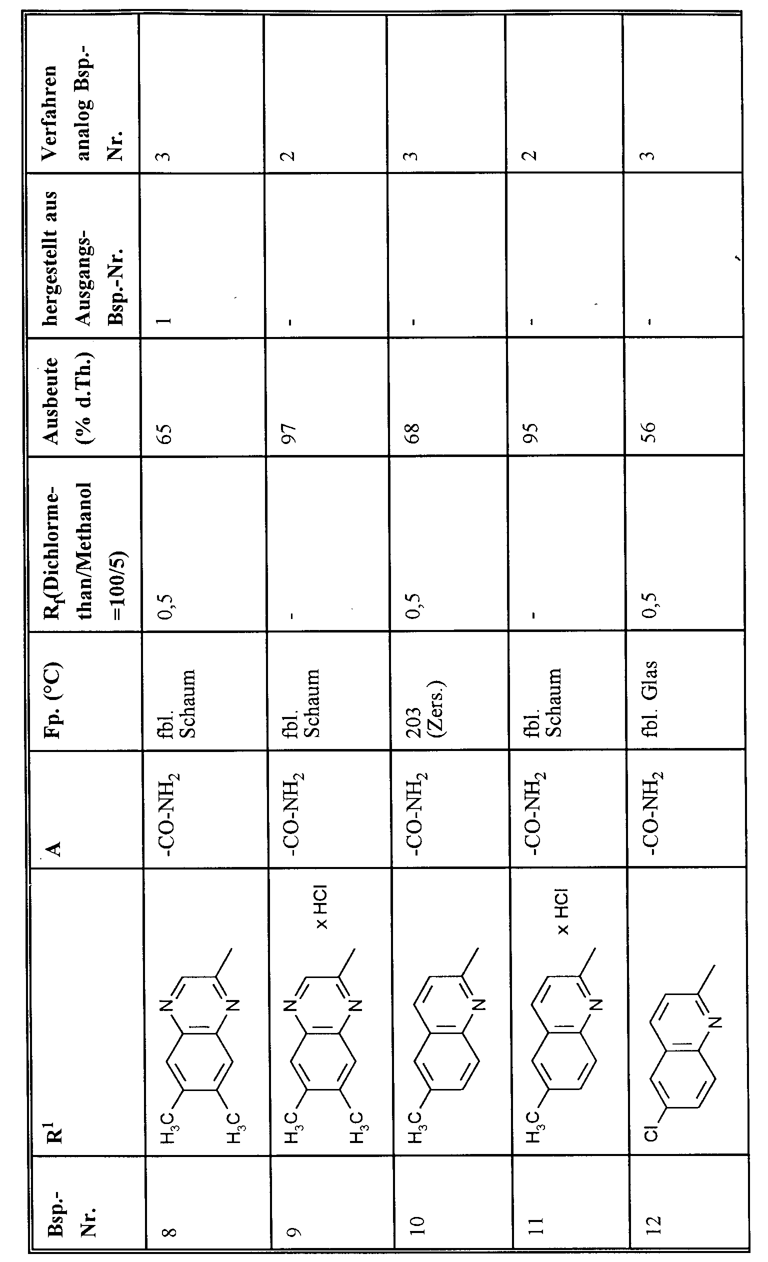

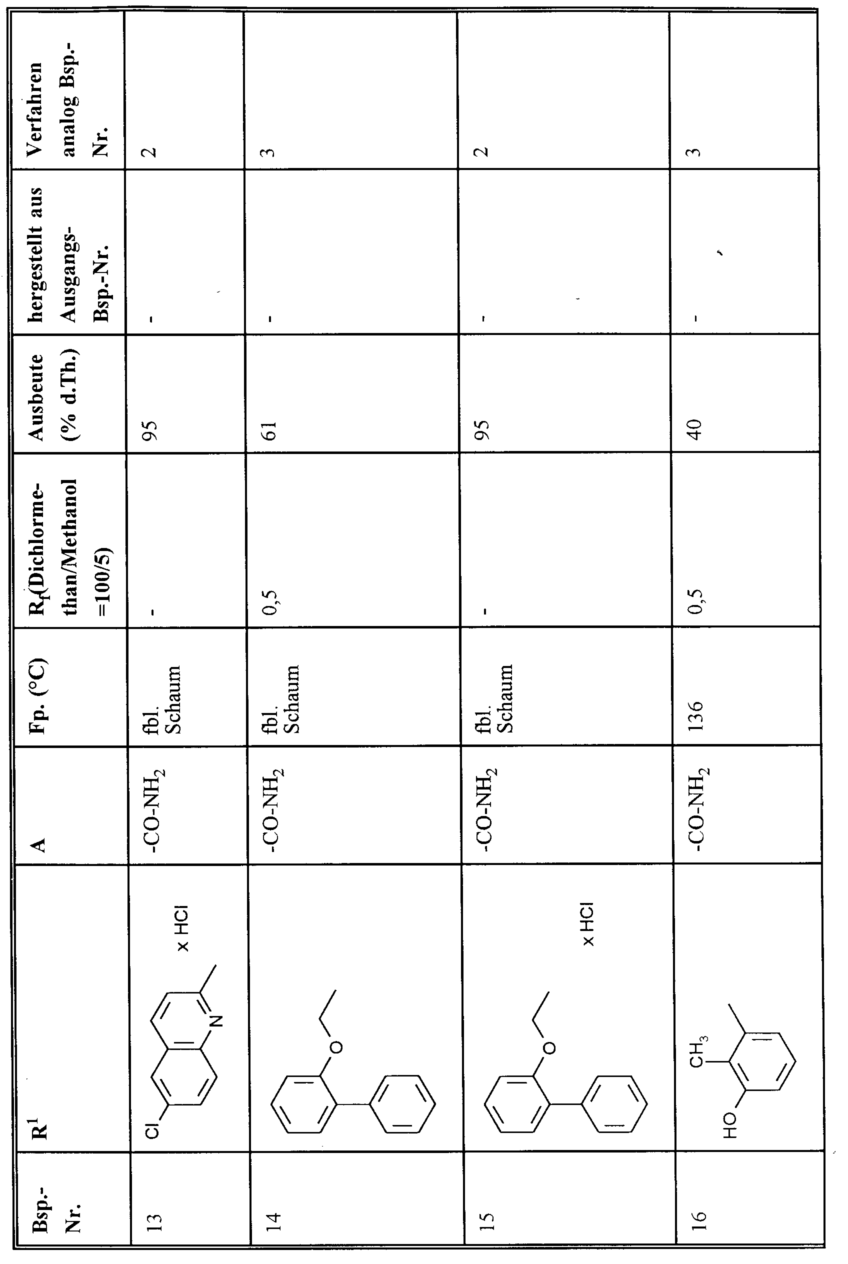

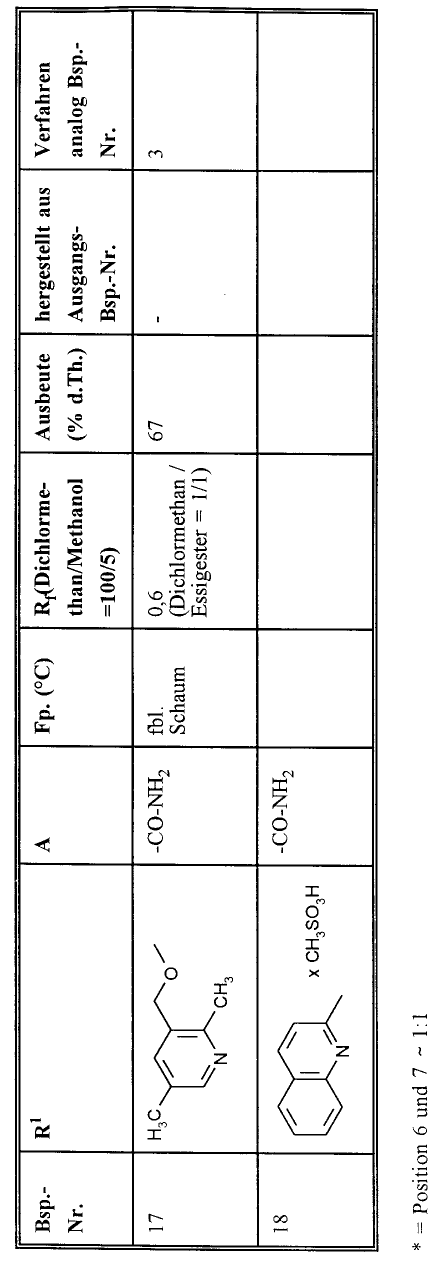

### Beispiel 19

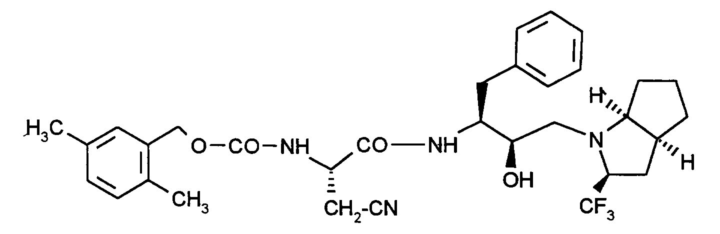

50 mg (0,08 mMol) der Verbindung aus Beispiel 3 werden in 2 ml Pyridin gelöst, unter Argonatmosphäre mit 100 mg (0,92 mMol) Dimethylcarbamoylchlorid versetzt und über Nacht im Ölbad (100°C) erhitzt. Nach dem Abkühlen wird alles im Vakuum zur Trockne eingedampft und der Rückstand mit 5 ml Wasser und 5 ml Dichlormethan ausgerührt.

Die organische Phase wird abgetrennt, getrocknet und zur Trockne eingedampft und der Rückstand säulenchromatographisch getrennt (Kieselgel 60, Laufmittel: Dichlormethan/Essigester = 1/1).

Farbloser Schaum, Ausbeute: 26 mg (53,6 % der Theorie)
R_{f} = 0,8 (Dichlormethan/Essigester = 1/1).

## Patentansprüche

1. Pseudopeptide mit trifluoromethyl-substituiertem 2-Azabicyclooctan der allgemeinen Formel (I) 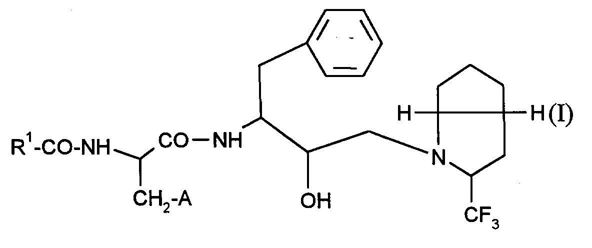 in welcher
A für Cyano oder für einen Rest der Formel -CO-NH₂ steht,
R¹ für einen Rest der Formel 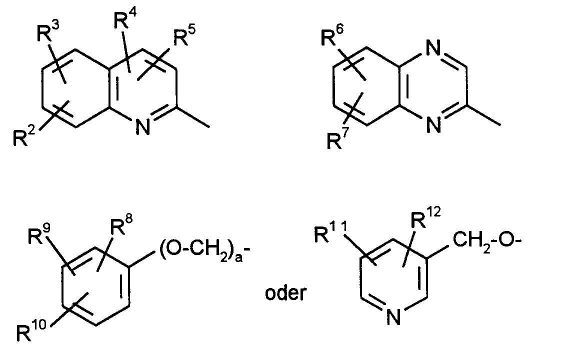 steht,
worin
R², R³, R⁴, R⁵, R⁶, R⁷, R⁸, R⁹, R¹⁰, R¹¹ und R¹² gleich oder verschieden sind und Phenyl, Hydroxyl, Wasserstoff, Halogen, geradkettiges oder verzweigtes Alkyl mit bis zu 4 Kohlenstoffatomen, Morpholinyl, Phenylthio oder Cycloalkyl mit 3 bis 6 Kohlenstoffatomen bedeuten,
a eine Zahl 0 oder 1 bedeutet,
und deren Salze.

2. Verbindungen der allgemeinen Formel (I), gemäß Anspruch 1
in welcher
A für Cyano oder für einen Rest der Formel -CO-NH₂ steht,
R¹ für einen Rest der Formel 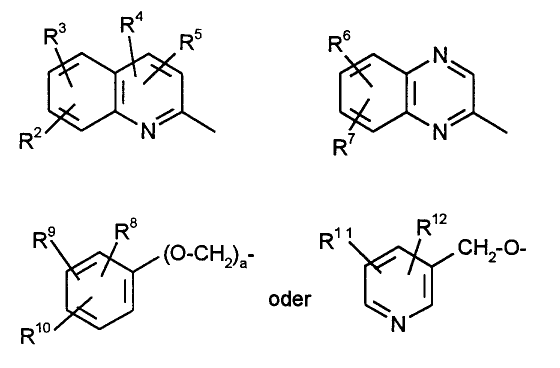 steht,
worin
R², R³, R⁴, R⁵, R⁶, R⁷, R⁸, R⁹, R¹⁰, R¹¹ und R¹² gleich oder verschieden sind und Phenyl, Hydroxyl, Wasserstoff, Fluor, Chlor, Brom, geradkettiges oder verzweigtes Alkyl mit bis zu 3 Kohlenstoffatomen, Morpholinyl, Phenylthio, Cyclopentyl oder Cyclohexyl bedeuten,
a eine Zahl 0 oder 1 bedeutet,
und deren Salze.

3. Verbindungen der allgemeinen Formel (I), gemäß Anspruch 1
in welcher
A für Cyano oder für einen Rest der Formel -CO-NH₂ steht,
R¹ für einen Rest der Formel 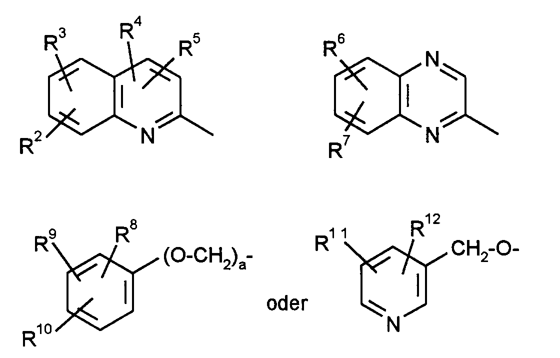 steht,
worin
R², R³, R⁴, R⁵, R⁶, R⁷, R⁸, R⁹, R¹⁰, R¹¹ und R¹² gleich oder verschieden sind Wasserstoff, Phenyl, Hydroxyl, Fluor, Chlor, Brom, Methyl, Phenylthio, Cyclopentyl, Cyclohexyl oder Morpholinyl bedeuten,
a eine Zahl 0 oder 1 bedeutet,
und deren Salze.

4. Verfahren zur Herstellung der Verbindungen der allgemeinen Formel (I), gemäß Anspruch 1, dadurch gekennzeichnet, daß man
Carbonsäuren der allgemeinen Formel (II)
R¹-CO₂-H (II)
in welcher
R¹ die im Anspruch 1 angegebene Bedeutung hat
mit der Verbindung der Formel (III), 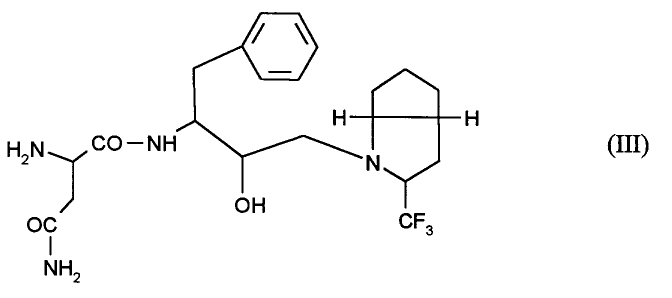 gegebenenfalls unter vorgeschalteter Aktivierung der Carbonsäure, in inerten Lösemitteln und in Anwesenheit einer Base und/oder eines Hilfsmittels umsetzt,
und im Fall A = CN abschließend mit Dimethylcarbamoylchlorid, ebenfalls in einem der oben aufgeführten Lösemittel umsetzt.

5. Arzneimittel enthaltend eine oder mehrere Verbindungen gemäß Anspruch 1.
